**Europäisches Patentamt**

**European Patent Office**

**Office européen des brevets**

(11) Veröffentlichungsnummer: **0 249 822 B1**

(12) # EUROPÄISCHE PATENTSCHRIFT

(45) Veröffentlichungstag der Patentschrift: **16.10.91**

(51) Int. Cl.⁵: **A61N 1/36**, A61B 5/00

(21) Anmeldenummer: **87108115.4**

(22) Anmeldetag: **04.06.87**

(54) Frequenzgesteuerter Herzschrittmacher.

(30) Priorität: **16.06.86 DE 3620278**

(43) Veröffentlichungstag der Anmeldung:
**23.12.87 Patentblatt 87/52**

(45) Bekanntmachung des Hinweises auf die
Patenterteilung:
**16.10.91 Patentblatt 91/42**

(84) Benannte Vertragsstaaten:
**DE FR GB IT NL SE**

(56) Entgegenhaltungen:
**EP-A- 0 133 828**
**WO-A-85/05279**
**FR-A- 2 225 060**

(73) Patentinhaber: **SIEMENS AKTIENGESELL-
SCHAFT**
**Wittelsbacherplatz 2**
**W-8000 München 2(DE)**

(72) Erfinder: **Wirtzfeld, Alexander, Prof. Dr.**
**Schultheissstrasse 35B**
**W-8070 Ingolstadt/Friedrichshofen(DE)**
Erfinder: **Stangl, Karl, Dr.**
**Landwehrstrasse 7**
**W-8000 München 2(DE)**
Erfinder: **Heinze, Roland, Dipl.-Ing.**
**Simbacher Strasse 9**
**W-8000 München 80(DE)**

## Beschreibung

Die Erfindung bezieht sich auf einen frequenzgesteuerten Herzschrittmacher gemäß Oberbegriff des Patentanspruchs 1.

Ein derartiger Herzschrittmacher ist aus der WO 85/05279 bekannt. Dort wird ein Herzschrittmacher beschrieben, bei dem die Stimulationsfolgefrequenz in Abhängigkeit von der durch einen Temperatursensor erfaßten Bluttemperatur gesteuert wird. Ferner enthält diese Patentanmeldung einen Hinweis, daß die Bluttemperatur und die daraus ausgewerteten Resultate dazu verwendet werden können, die Effektivität eines Herzschrittmachers zu kontrollieren, der als Steuerparameter nicht die zentralvenöse Bluttemperatur benutzt, sondern z.B. die zentralvenöse Sauerstoffsättigung, die Atmungsfrequenz und dergleichen. Es ist jedoch nicht angegeben, wie eine gleichzeitige Auswertung von Bluttemperatur und Blutsauerstoffsättigung durchgeführt werden könnte.

Bei höherer Belastung gerät der Körper bekanntlich in die anaerobe Stoffwechselsituation, in der sich die Änderungen des Blutsauerstoffs asymptotisch dem Nullwert nähern.

Aufgabe vorliegender Erfindung ist es, einen frequenzgesteuerten Herzschrittmacher der eingangs genannten Art dahingehend weiterzubilden, daß die Genauigkeit der Frequenzsteuerung von einer Absenkung des Blutsauerstoffs bei höherer Belastung unbeeinflußt bleibt.

Die Aufgabe wird erfindungsgemäß durch die kennzeichnenden Merkmale des Patentanspruchs 1 gelöst.

Die Erfindung nutzt die Tatsache aus, daß sich die Temperatur im venösen Blut bei Belastung erhöht. Damit verläuft das Temperatursignal bei höherer Belastung in umgekehrter Weise wie das blutsauerstoffabhängige Signal. Das mit höherer Belastung absinkende blutsauerstoffabhängige Signal wird gemäß der Erfindung durch das mit höherer Belastung ansteigende Temperatursignal weiter virtuell abgesenkt. Es ergibt sich damit ein Steuersignal für den Frequenzsteuerteil des frequenzgesteuerten Herzschrittmachers, das exakter ist als das Steuersignal bei herkömmlichen frequenzgesteuerten Herzschrittmachern.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Es zeigen:

Figur 1 ein erstes Ausführungsbeispiel des erfindungsgemäßen frequenzgesteuerten Herzschrittmachers im Prinzipschaltbild,

Figur 2 ein zweites Ausführungsbeispiel des erfindungsgemäßen frequenzgesteuerten Herzschrittmachers im Prinzipschaltbild und

Figur 3 die Blutsauerstoff/Temperaturmeßeinrichtung des Herzschrittmachers der Figur 2 in detaillierterer Darstellung.

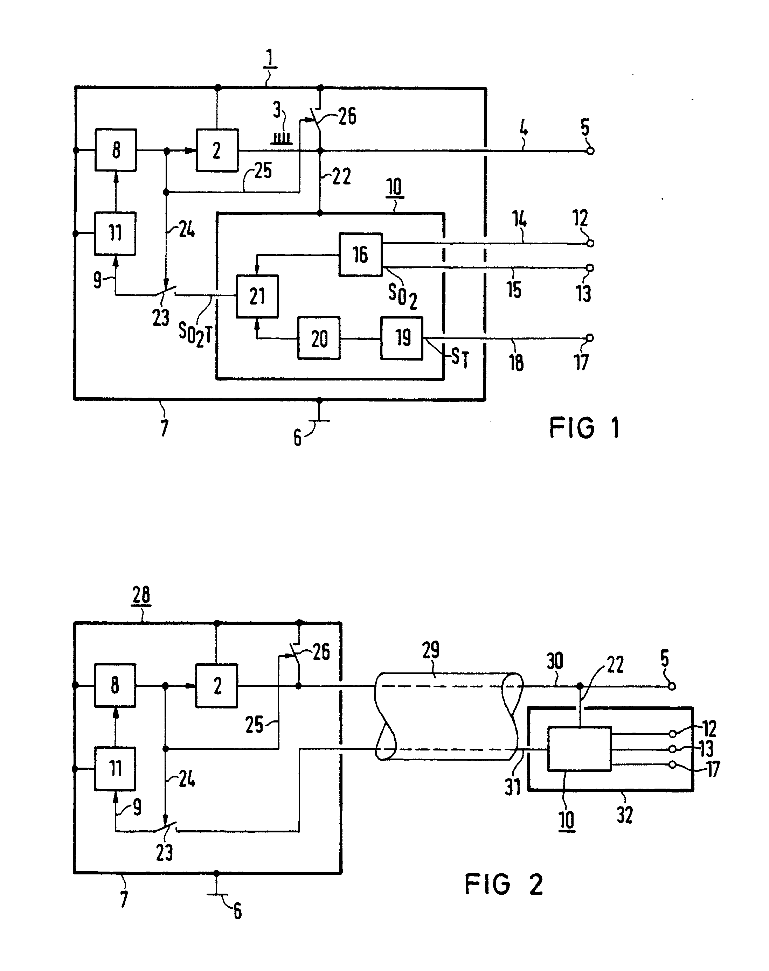

Der frequenzgesteuerte Herzschrittmacher 1 gemäß Figur 1 umfaßt einen Pulsgenerator 2 für Stimulationsimpulse 3, die über einen Stimulationskatheter 4 der Stimulationselektrode 5 zuleitbar sind. Die indifferente Elektrode des Herzschrittmachers ist mit der Kennziffer 6 angedeutet. Sie entspricht dem leitenden (z.B. metallischen) Gehäuse 7 des Herzschrittmachers 1. Der Herzschrittmacher 1 beinhaltet auch noch einen Frequenzsteuerteil 8 zur Einstellung einer erwünschten Stimulationsfrequenz am Pulsgenerator 2.

Der Frequenzsteuerteil 8 wird über eine Steuerleitung 9 von dem Ausgangssignal

$$S_{O_2T}$$

einer Blutsauerstoff/Temperaturmeßeinrichtung 10 invers gesteuert. Zu diesem Zwecke ist in die Steuerleitung 9 zwischen dem Frequenzsteuerteil 8 und der Blutsauerstoff/Temperaturmeßeinrichtung 10 ein Invertierglied 11 eingeschaltet. Mit größer werdendem Ausgangssignal

$$S_{O_2T}$$

wird also die Stimulationsfrequenz am Pulsgenerator 2 niedriger. Bei kleiner werdendem Ausgangssignal

$$S_{O_2T}$$

wird die Stimulationsfrequenz entsprechend erhöht.

Die Blutsauerstoff/Temperaturmeßeinrichtung 10 umfaßt für die Blutsauerstoffmessung einen Lichtsender 12 und einen Lichtempfänger 13, die über Verbindungsleitungen 14 und 15 mit einem Verstärker 16 für das blutsauerstoffabhängige Signal

$$S_{O_2}$$

verbunden ist. Sie beinhaltet ferner einen Temperatursensor 17, der über eine Verbindungsleitung 18 an einem Verstärker 19 für das Temperatursignal $S_T$ angeschaltet ist. Dem Verstärker 19 für das Temperatursignal $S_T$ ist ein Invertierglied 20 nachgeschaltet. Das Ausgangssignal (invertiertes Temperatursignal) des Invertiergliedes 20 wird zusammen mit dem Ausgangssignal (blutsauerstoffabhängiges Signal) des Verstärkers 16 einer Überlagerstufe 21 zugeführt. In der Überlagerstufe 21 wird dem blutsauerstoffabhängigen Signal das invertierte Temperatursignal im Sinne einer Absenkung des blutsauerstoffabhängigen Signals im höheren Belastungsbereich überlagert. Während der Zeitdauer einer Blutsauerstoffmessung (intermittierend zwischen jeweils zwei aufeinanderfolgenden Stimulationsimpulsen) stellt die Leitung des Stimulationskatheters 4 die zweite Versorgungsleitung für die Blutsauerstoff/Temperaturmeßeinrichtung 10 dar. Zu diesem Zwecke ist die Blutsauerstoff/Temperaturmeßeinrichtung 10 mittels Verbindungsleitung 22 mit der Leitung des Stimulationskatheters 4 verbunden. Zum Zwecke der intermittierenden Blutsauerstoff/Temperaturmessung wird ein Schalter 23, der in der Steuerleitung 9 zwischen der Blutsauerstoff/Temperaturmeßeinrichtung 10 und dem Invertierglied 9 angeordnet ist, über eine Schaltleitung 24 vom Ausgangssignal des Frequenzsteuerteils 8 in dem Sinne gesteuert, daß er jeweils zwischen zwei aufeinanderfolgenden Stimulationsimpulsen 3 geschlossen wird. Gleichzeitig wird über die abgezweigte Schaltleitung 25 ein weiterer Schalter 26 geschlossen, wodurch die Stimulationselektrode 5 über den Stimulationskatheter 4 an Masse gelegt wird. Dadurch wird sichergestellt, daß kein Meßstrom über den Stimulationskreis fließen kann.

Der frequenzgesteuerte Herzschrittmacher 28 der Figur 2 unterscheidet sich von jenem der Figur 1 im wesentlichen dadurch, daß die Blutsauerstoff/Temperaturmeßeinrichtung 10 aus dem Gehäuse 7 des Herzschrittmachers ausgelagert ist. Vorzugsweise sitzt sie im Stimulationskatheter hinter der Stimulationselektrode 5. Zu diesem Zwecke ist der Stimulationskatheter als Bipolarkatheter ausgebildet, wie in der Figur 2 gestrichelt und durch die Kennziffer 29 angedeutet ist. Die erste Zuleitung des Bipolarkatheters 29, an der die Stimulationselektrode 5 angedeutet ist, ist in der Figur 2 mit 30 bezeichnet. Die zweite Zuleitung, in der die Sonderkapsel 32 mit der Blutsauerstoff/Temperaturmeßeinrichtung 10 samt Lichtsender 12, Lichtempfänger 13 und Temperatursensor 17 eingebaut ist, ist in der Figur 2 mit 31 angedeutet. Ansonsten funktioniert der Herzschrittmacher der Figur 2 genauso wie im Zusammenhang mit dem Herzschrittmacher der Figur 1 beschrieben wurde.

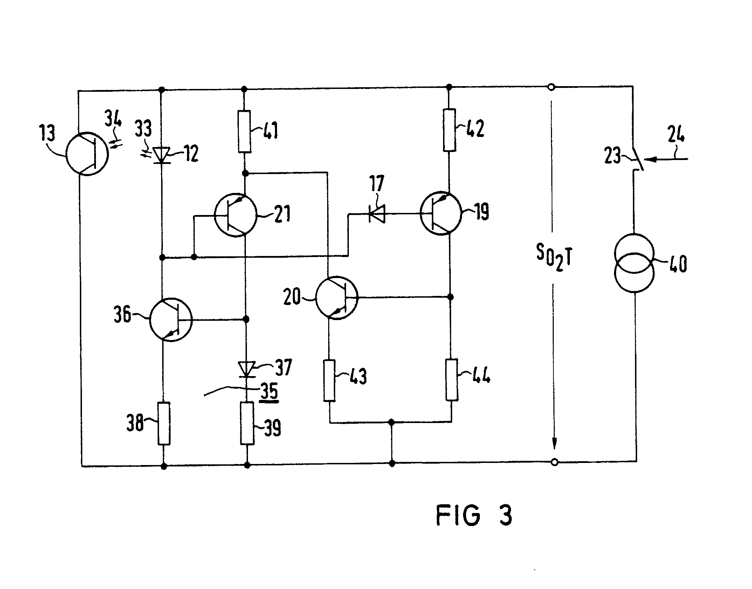

Die Figur 3 zeigt die Blutsauerstoff/Temperaturmeßeinrichtung 10 des Herzschrittmachers der Figur 2 in detaillierter Darstellung. Der Lichtsender 12 ist hier eine Leuchtdiode (LED). Der Lichtempfänger 13 ist ein Fototransistor. Das vom Lichtsender 12 ins Blut abgestrahlte Licht ist mit der Kennziffer 33 angedeutet. Das vom Lichtempfänger 13 nach Reflexion am Blut empfangene Licht ist mit der Kennziffer 34 angedeutet. Als Temperatursensor 17 dient ein temperaturabhängiger Halbleiter. Als Verstärker 19 für das Temperatursignal ist ein Transistor verwendet. Das Invertierglied 20 wird ebenfalls durch einen Transistor gebildet. Das invertierte Ausgangssignal gelangt vom Kollektor des Transistors 20 zum Kollektor eines der Überlagerstufe 21 bildenden Stromsteuertransistors. Der Stromsteuertransistor verändert die Stromversorgung 35 für den Lichtsender 12 (LED) in dem Sinne, daß eine ansteigende Temperatur einen verringerten Stromfluß durch die LED bewirkt, wodurch ein abfallender Sauerstoffgehalt im Blut simuliert wird. Die Stromversorgung 35 umfaßt einen Transistor 36, eine Stabilisierungsdiode 37 und ohm'sche Widerstände 38 und 39. Mit 40 ist die Konstantstromquelle für die Versorgung des gesamten Systems bezeichnet. Die Bauelemente 41 bis 44 sind wieder ohm'sche Widerstände.

**Patentansprüche**

1. Frequenzgesteuerter Herzschrittmacher (1,28), dessen Stimulationsfrequenz in Abhängigkeit von einem mit einem Blutsauerstoffsensor (12, 13) erzeugten blutsauerstoffabhängigen Signal und in Abhängigkeit von einem mit einem Temperatursensor (17) erzeugten bluttemperaturabhängigen Signal ($S_T$) gesteuert wird, **durch gekennzeichnet, daß** dem Blutsauerstoffsensor (12,13) der Temperatursensor (17) zugeordnet ist, wobei mit dem temperaturabhängigen Signal das blutsauerstoffabhängige Signal ($S_{O2}$) des Blutsauerstoffsensors im Sinne einer Absenkung des Blutsauerstoffsignals im höheren Belastungsbereich überlagert wird.

2. Herzschrittmacher nach Anspruch 1, **dadurch gekennzeichnet,** daß das invertierte temperaturabhängige Signal zum blutsauerstoffabhängigen Signal addiert wird.

3. Herzschrittmacher nach Anspruch 1 oder 2, **dadurch gekennzeichnet,** daß der Blutsauerstoffsensor (12, 13) und der Temperatursensor (17) in einem Stimulationskatheter (29) für die Stimulationselektrode (5) angeordnet sind.

4. Herzschrittmacher nach Anspruch 3, **dadurch gekennzeichnet,** daß der Blutsauerstoffsensor (12, 13) und der Temperatursensor (17) in einer Sonderkapsel (32) im Stimulationskatheter angeordnet sind.

5. Herzschrittmacher nach einem der Ansprüche 1 bis 4, **dadurch gekennzeichnet,** daß der Sauerstoffsensor (12, 13) einen Lichtsender (12) zur Abstrahlung von Licht (33) in das Blut und einen Lichtempfänger (13) zum Empfangen des am Blut reflektierten Lichtes (34) umfaßt und daß der Temperatursensor (17) den Betriebsstrom des Lichtsenders (12) im Sinne einer Absenkung des Betriebsstromes bei steigender Temperatur beeinflußt, wodurch ein abfallender Sauerstoffgehalt im Blut simuliert wird.

## Claims

1. Frequency-controlled cardiac pacemaker (1, 28), the stimulation frequency of which is controlled as a function of a signal, which is generated with a blood-oxygen sensor (12, 13) and is dependent upon the oxygen contained in the blood, and as a function of a signal ($S_T$) which is generated with a temperature sensor (17) and is dependent upon the blood temperature, characterised in that the temperature sensor (17) is associated with the blood-oxygen sensor (12, 13), the signal ($S_{O2}$) of the blood-oxygen sensor, which is dependent upon the oxygen contained in the blood, being superimposed with the temperature-dependent signal for the purposes of lowering the blood-oxygen signal in the higher load range.

2. Cardiac pacemaker according to claim 1, characterised in that the inverted temperature-dependent signal is added to the signal dependent upon the oxygen contained in the blood.

3. Cardiac pacemaker according to claim 1 or 2, characterised in that the blood-oxygen sensor (12, 13) and the temperature sensor (17) are arranged in a stimulation catheter (29) for the stimulation electrode (5).

4. Cardiac pacemaker according to claim 3, characterised in that the blood-oxygen sensor (12, 13) and the temperature sensor (17) are arranged in a probe capsule (32) in the stimulation catheter.

5. Cardiac pacemaker according to one of the claims 1 to 4, characterised in that the oxygen sensor (12, 13) comprises a light transmitter (12) for the purpose of radiating light (33) into the blood and a light receiver (13) for receiving the light (34) reflected by the blood and in that the temperature sensor (17) affects the operating current of the light transmitter (12) for the purposes of lowering the operating current given rising temperature, whereby a falling oxygen content in the blood is simulated.

## Revendications

1. Stimulateur cardiaque (1,28) à fréquence commandée et dont la fréquence de stimulation est commandée en fonction d'un signal qui dépend de l'oxygène dans le sang et qui est produit par un détecteur (12,13) de l'oxygène dans le sang, et en fonction d'un signal ($S_T$) qui dépend de la température du sang et qui est produit par un capteur de température (17), caractérisé par le fait que le capteur de température (17) est associé au détecteur (12,13) de l'oxygène dans le sang, le signal ($S_{O2}$), qui dépend de l'oxygène dans le sang, et qui est délivré par le détecteur de l'oxygène dans le sang, est superposé au signal qui dépend de la température, dans le sens d'une réduction du signal de l'oxygène dans le sang dans la plage supérieure de charge.

2. Stimulateur cardiaque suivant la revendication 1, caractérisé par le fait que le signal, qui dépend de la température, est ajouté à l'état inversé au signal qui dépend de l'oxygène dans le sang.

3. Stimulateur cardiaque suivant la revendication 1 ou 2, caractérisé par le fait que le détecteur (12,13) de l'oxygène dans le sang et le capteur de température (17) sont disposés dans un cathéter de stimulation (29) prévu pour l'électrode de stimulation (5).

4. Stimulateur cardiaque suivant la revendication 3, caractérisé par le fait que le détecteur (12,13) de l'oxygène dans le sang et le capteur de température (17) sont disposés dans une capsule de sonde (32) dans le cathéter de stimulation.

5. Stimulateur cardiaque suivant l'une des revendications 1 à 4, caractérisé par le fait que le détecteur d'oxygène (12,13) comprend une source de lumière (12) servant à émettre une lumière (33) dans le sang et un récepteur de lumière (13) servant à recevoir la lumière (34) réfléchie sur le sang, et que le capteur de température (17) influe sur le courant de service de la source de lumière (12) en vue de réduire ce courant lorsque la température augmente, ce qui simule une diminution de la teneur de l'oxygène dans le sang.

FIG 1

FIG 2

FIG 3